(19) **Europäisches Patentamt**

**European Patent Office**

**Office européen des brevets**

(11) **EP 1 189 577 B1**

(12) **EUROPÄISCHE PATENTSCHRIFT**

(45) Veröffentlichungstag und Bekanntmachung des Hinweises auf die Patenterteilung:
**18.08.2004 Patentblatt 2004/34**

(21) Anmeldenummer: **00942103.3**

(22) Anmeldetag: **20.06.2000**

(51) Int Cl.[7]: **A61K 7/00**

(86) Internationale Anmeldenummer:
**PCT/EP2000/005667**

(87) Internationale Veröffentlichungsnummer:
**WO 2001/000143 (04.01.2001 Gazette 2001/01)**

(54) **KOSMETISCHE EMULSIONEN**

COSMETIC EMULSIONS

EMULSIONS COSMETIQUES

(84) Benannte Vertragsstaaten:
**DE ES FR GB IT**

(30) Priorität: **29.06.1999 DE 19929505**

(43) Veröffentlichungstag der Anmeldung:
**27.03.2002 Patentblatt 2002/13**

(73) Patentinhaber: **Cognis Deutschland GmbH & Co. KG**
**40589 Düsseldorf (DE)**

(72) Erfinder:
• **LE HEN FERRENBACH, Catherine**
**F-77100 Meaux (FR)**

• **MORAND, Corinne**
**F-77100 Meaux (FR)**
• **BRÜNING, Stefan**
**D-40597 Düsseldorf (DE)**
• **ANSMANN, Achim**
**D-40699 Erkrath (DE)**
• **CAPITO, Marco**
**D-40599 Düsseldorf (DE)**

(56) Entgegenhaltungen:
DE-A- 19 726 785          DE-A- 19 821 402
US-A- 5 807 561

EP 1 189 577 B1

## Beschreibung

### Gebiet der Erfindung

Die Erfindung befindet sich auf dem Gebiet der Kosmetik und betrifft besonders feinteilige, lagerstabile Emulsionen, die ein temäres bzw. quaternäres Emulgatorsystem enthalten.

### Stand der Technik

Im Bereich kosmetischer Emulsionen für die Haut- und Haarpflege werden vom Verbraucher eine Vielzahl von Anforderungen gestellt: abgesehen von den reinigenden und pflegenden Effekten, die den Anwendungszweck bestimmen, wird Wert auf so unterschiedliche Parameter wie höchstmögliche dermatologische Verträglichkeit, elegantes Erscheinungsbild, optimaler sensorischer Eindruck und Lagerstabilität gelegt. Einige dieser Merkmale, wie z.B. die Hautverträglichkeit lassen sich vom Kosmetikchemiker weitgehend objektiv bestimmen, kommt es jedoch zum sensorischen Eindruck so ist die Beurteilung durch Probanden letztlich immer subjektiv, auch wenn eine statistische Auswertung wenigstens zu einer Signifikanz führt. Unabhängig von diesen Faktoren ist jedoch bekannt, daß die Feinteiligkeit einer Emulsion in direktem Zusammenhang sowohl mit ihrem äußeren Erscheinungsbild als auch der Lagerstabilität steht. Demzufolge besteht ein lebhaftes Interesse daran, Emulsionen zur Verfügung zu stellen, die sich durch besondere Feinteiligkeit auszeichnen und auch bei Temperaturbelastung keine Tendenz zur Agglomeration der Tröpfchen oder gar zur Phasentrennung zeigen. In diesem Zusammenhang sei auf die Aufsätze von A.Ansmann **[Seifen-Öle-Fette-Wachse, 117, 518 (1991)],** C.Cabeta **[SÖFW-Journal, 120, 162 (1994)],** P.Hameyer **[SÖFW-Journal, 121, 216, (1995)]** und insbesondere A.Wadle **[Parf.Kosm. 77, 250 (1996)]** verwiesen.

Als besonders vorteilhaft zur Herstellung feinteiliger Emulsionen hat sich die PIT-Methode erwiesen. Bei der einstufigen Methode werden üblicherweise die Emulsionskomponenten bei Raumtemperatur vorgelegt und gemeinsam auf etwa 80 °C erwärmt, wobei der lamellare flüssigkristalline Phasenbereich durchlaufen wird. Nach Abkühlen auf Raumtemperatur erhält man eine feinteilig emulgierte Ölphase. Beim zweistufigen heiß/heiß-Verfahren, das im technischen Bereich bevorzugt Anwendung findet, wird die heiße, wasserfreie Phase aus Ölkörper und Emulgator mit einem Teil der Wassermenge bei gleicher Temperatur emulgiert. Die Emulsion durchläuft dabei im Emulsionskonzentrat eine transparente Mikroemulsionsphase, der bei etwa 85 °C die restliche Wassermenge hinzugegeben wird. Hierdurch invertiert die Mikroemulsion zu einer ebenfalls sehr feinteiligen O/W-Emulsion.

Obschon dem Fachmann also sehr wohl Maßnahmen bekannt sind, mit deren Hilfe er grundsätzlich zu feinteiligen Emulsionen gelangen kann, sind die Emulsionen des Stands der Technik - bedingt durch die Auswahl der eingesetzten Emulgatoren - nach wie vor nicht völlig zufriedenstellend. Insbesondere das äußere Erscheinungsbild, der sensorische Eindruck und die Temperaturlagerbeständigkeit gilt es zu verbessern. Demzufolge hat die Aufgabe der vorliegenden Erfindung darin bestanden, neue Emulsionen zur Verfügung zu stellen, welche sich gegenüber dem Stand der Technik gleichzeitig durch verbesserte Feinteiligkeit und Lagerstabilität, insbesondere bei höheren Temperaturen auszeichnen.

### Beschreibung der Erfindung

Gegenstand der Erfindung sind kosmetische Emulsionen auf Basis von Wasser, Ölkörpern und einer Emulgatormischung, bei denen mindestens 60, vorzugsweise mindestens 70 und insbesondere mindestens 80 % der Tröpfchen einen mittleren Durchmesser kleiner 0,2 µm aufweisen, enthaltend

    (a) $C_{16}$-$C_{22}$-Alkyl- und/oder Alkenyloligoglykoside,
    (b) $C_{16}$-$C_{22}$-Fettalkohole und
    (c) $C_{22}$-Fettalkoholethoxylate.

Überraschenderweise wurde gefunden, daß sich unter Einsatz der genannten temären Emulgatormischung besonders feinteilige PIT- oder Mikroemulsionen erhalten lassen. Diese zeigen weiterhin den Vorteil einer hohen Lagerbeständigkeit, d.h. auch bei Temperaturbelastung kommt es weder zu einer Agglomeration der Tröpfchen noch zu einer Entmischung. Die Erfindung schließt die Erkenntnis ein, daß sich die anwendungstechnischen Eigenschaften, insbesondere die Feinteiligkeit und die sensorische Beurteilung weiter verbessern lassen, wenn man als weitere Emulgatorkomponente höher ethoxylierte Fettalkohole mitverwendet.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen $C_6$-$C_{22}$-Fettsäuren mit linearen $C_6$-$C_{22}$-Fettalkoholen, Ester von verzweigten $C_6$-$C_{13}$-Carbonsäuren mit linearen $C_6$-$C_{22}$-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen $C_6$-$C_{22}$-Fettsäuren mit verzweigten Alkoholen, insbesondere 2-Ethylhexanol, Ester von Hydroxycarbonsäuren mit linearen oder verzweigten $C_6$-$C_{22}$-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis $C_6$-$C_{10}$-Fettsäuren, flüssige Mono-/Di-/Triglyceridmischungen auf Basis von $C_6$-$C_{18}$-Fettsäuren, Ester von $C_6$-$C_{22}$-Fettalkoholen und/oder Guerbetalkoholen mit aromatischen Carbonsäuren, insbesondere Benzoesäure, Ester von $C_2$-$C_{12}$-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle bzw. ungesättigte Triglyceride, verzweigte primäre Alkohole, substituierte Cyclohexane, lineare und verzweigte $C_6$-$C_{22}$-Fettalkoholcarbonate, Guerbetcarbonate, Ester der Benzoesäure mit linearen und/oder verzweigten $C_6$-$C_{22}$-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestem mit Polyolen, Siliconöle und/oder aliphatische bzw. naphthenische Kohlenwasserstoffe, wie z.B. wie Squalan, Squalen oder Dialkylcyclohexane in Betracht.

Alkyl- und/oder Alkenyloligoglykoside

Alkyl- und Alkenyloligoglykoside, die als Komponente (a) in Frage kommen, stellen bekannte nichtionische Tenside dar, die vorzugsweise der Formel (I) folgen,

$$R^1O\text{-}[G]_p$$
$$(I)$$

in der $R^1$ für einen Alkyl- und/oder Alkenylrest mit 16 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Schriften **EP-A1 0 301 298** und **WO 90/03977** verwiesen. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside.** Die Indexzahl p in der allgemeinen Formel **(I)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest $R^1$ kann sich von primären Alkoholen mit 16 bis 22, vorzugsweise 16 bis 18 Kohlenstoffatomen ableiten. Typische Beispiele sind Cetylalkohol, Palmoleylalkohol, Stearylalkohol, Isostearylalkohol, Oleylalkohol, Elaidylalkohol, Arachidylalkohol, Gadoleylalkohol, Behenylalkohol und Erucylalkohol sowie deren technische Mischungen, wie sie beispielsweise bei der Hydrierung von technischen Fettsäuremethylestem erhalten werden. Besonders bevorzugt sind Alkyloligoglucoside der Formel (I), in der $R^1$ für einen Cetylstearyl- ("Cetearyl") oder einen Behenylrest steht, wobei der DP vorzugsweise im Bereich von 1 bis 3 liegt.

Fettalkohole

Unter Fettalkoholen, die die Komponente (b) darstellen sind primäre aliphatische Alkohole der Formel **(II)** zu verstehen,

$$R^2OH$$

**(II)**

in der $R^2$ für einen aliphatischen, linearen oder verzweigten Kohlenwasserstoffrest mit 16 bis 22 Kohlenstoffatomen und 0 und/oder 1, 2 oder 3 Doppelbindungen steht. Typische Beispiele sind Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen anfallen. Bevorzugt sind technische Fettalkohole mit 16 bis 18 Kohlenstoffatomen wie beispielsweise Palm- oder Talgfettalkohol bzw. der für kosmetische Zwecke häufig verwendete Cetearylalkohol, eine technische Mischung von Cetyl- und Stearylalkohol im ungefähren Gewichtsverhältnis 1 : 1. Ebenfalls bevorzugt wird Behenylalkohol eingesetzt. Vorzugsweise werden weiterhin solche Fettalkohole eingesetzt, deren Fettrest mit der Komponente (a) übereinstimmt. Zu diesem Zweck können in einer weiteren bevorzugten Ausführungsform auch technische Glykosid/Fettalkohol-Gemische eingesetzt werden, die gegebenenfalls durch Destillation oder Extraktion auf die gewünschte Alkoholmenge abgereichert werden. Derartige Mischungen sind unter der Bezeichnung Emulgade® PL 68/50 (Henkel) oder Montanov® 68 (SEPPIC) im Handel erhältlich. Das Gewichtsverhältnis zwischen den beiden Komponenten (a) und (b) kann dabei im Bereich 10 : 90 bis 90 : 10, vorzugsweise 25 : 75 bis 75 : 25 und insbesondere 40 : 60 bis 60 : 40 liegen. Besonders bevorzugt ist eine Mischung von ungefähr 50 : 50.

Fettalkoholethoxylate

Fettalkoholethoxylate, die als Komponente (c) in Frage kommen, stellen Anlagerungsprodukte von Ethylenoxid an primäre Alkohole dar, die vorzugsweise der Formel **(III)** folgen,

$$R^3O(CH_2CH_2O)_nH$$

**(III)**

in der $R^3$ für einen Behenylrest und n für Zahlen von 1 bis 15 steht. Die Ethoxylate werden nach den hinreichend bekannten Verfahren des Stands der Technik hergestellt. Herstellungsbedingt können sie eine konventionell breite oder auch eingeengte Homologenverteilung aufweisen. Besonders bevorzugt ist der Einsatz von Anlagerungsprodukten von durchschnittlich 8 bis 12 und insbesondere 10 Mol Ethylenoxid an technischen Behenylalkohol. Das Gewichtsverhältnis der Summe aus den Komponenten (a) und (b) zu (c) liegt in der Regel im Bereich 25 : 75 bis 75 : 25 und vorzugsweise 40 : 60 bis 60 : 40.

Hydrophile Fettalkoholethoxylate

In einer weiteren bevorzugten Ausführungsform der Erfindung können die Emulsionen als fakultative Komponente (d) weitere Fettalkoholethoxylate enthalten, welche sich von denen der Gruppe (c) dadurch unterscheiden, daß sie einen höheren Ethoxylierungsgrad aufweisen; sie werden daher im folgenden zur Abgrenzung als "hydrophile" Fettalkoholethoxylate bezeichnet, die vorzugsweise der Formel **(IV)** folgen,

$$R^4O(CH_2CH_2O)_mH$$

**(IV)**

in der $R^4$ für einen linearen oder verzweigten Alkyl- und/oder Alkenylrest mit 6 bis 22 Kohlenstoffatomen und m für Zahlen von 15 bis 35 steht. Die Herstellung derartiger nichtionischer Tenside entspricht dem oben ausgeführten. Typische Beispiele sind Anlagerungsprodukte von durchschnittlich 20 bis 25 Mol Ethylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Besonders bevorzugt sind die Addukte von 20 bzw. 25 Mol Ethylenoxid an Cetearylalkohol oder Behenylalkohol. Das Gewichtsverhältnis zwischen den Fettalkoholethoxylaten der Gruppen (c) und (d) liegt vorzugsweise im Bereich 1 : 1 bis 5 : 1 und insbesondere 2 : 1 bis 3 : 1.

Emulsionen

Die erfindungsgemäßen Emulsionen können die temäre bzw. quatemäre Emulgatormischung in Mengen von 1 bis 25, vorzugsweise 2 bis 10 und insbesondere 5 bis 8 Gew.-% - bezogen auf die Mittel - enthalten. Vorzugsweise setzen sich die erfindungsgemäßen Emulsionen wie folgt zusammen:

> (a) 1 bis 50, vorzugsweise 5 bis 20 Gew.-% Ölkörper und
> (b) 1 bis 25, vorzugsweise 2 bis 10 Gew.-% Emulgatormischung,

mit der Maßgabe, daß sich die Mengenangaben mit Wasser und gegebenenfalls weiteren üblichen Hilfs- und Zusatzstoffen zu 100 Gew.-% ergänzen. Besonders bevorzugt sind Emulsionen, die folgende Zusammensetzung aufweisen:

> (a) 1 bis 50, vorzugsweise 5 bis 20 Gew.-% Ölkörper und
> (b1) 1 bis 5, vorzugsweise 2 bis 4 Gew.-% $C_{16}$-$C_{22}$-Alkyl- und/oder Alkenyloligoglykoside,
> (b2) 1 bis 5, vorzugsweise 2 bis 4 Gew.-% $C_{16}$-$C_{22}$-Fettalkohole,
> (b3) 1 bis 5, vorzugsweise 2 bis 4 Gew.-% $C_{22}$-Fettalkoholethoxylate und
> (b4) 0 bis 4, vorzugsweise 1 bis 2 Gew.-% hydrophile Fettalkoholethoxylate

mit der Maßgabe, daß sich die Mengenangaben mit Wasser und gegebenenfalls weiteren üblichen Hilfs- und Zusatzstoffen zu 100 Gew.-% ergänzen.

**Gewerbliche Anwendbarkeit**

Die erfindungsgemäßen Emulsionen können zur Herstellung von Zubereitungen, wie beispielsweise Haarshampoos, Haarlotionen, Schaumbäder, Duschbäder, Cremes, Gele, Lotionen, alkoholische und wäßrig/alkoholische Lösungen, Emulsionen, Wachs/ Fett-Massen, Stiftpräparaten, Pudem oder Salben dienen. Diese Mittel können ferner als weitere Hilfs- und Zusatzstoffe milde Tenside, Co-Emulgatoren, Überfettungsmittel, Perlglanzwachse, Konsistenzgeber, Verdickungsmittel, Polymere, Siliconverbindungen, Fette, Wachse, Stabilisatoren, biogene Wirkstoffe, Deodorantien, Antitranspirantien, Antischuppenmittel, Filmbildner, Quellmittel, UV-Lichtschutzfaktoren, Antioxidantien, Hydrotrope, Konservierungsmittel, Insektenrepellentien, Selbstbräuner, Tyrosininhibitoren (Depigmentierungsmittel), Solubilisatoren, Parfümöle, Farbstoffe und dergleichen enthalten.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, $\alpha$-Olefinsulfonate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Co-Emulgatoren** kommen beispielsweise nichtionogene Tenside aus mindestens einer der folgenden Gruppen in Frage:

> ➢ Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
> ➢ Anlagerungsprodukte von 1 bis 15 Mol Ethylenoxid an Ricinusöl und/oder gehärtetes Ricinusöl;
> ➢ Anlagerungsprodukte von 15 bis 60 Mol Ethylenoxid an Ricinusöl und/oder gehärtetes Ricinusöl;
> ➢ Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
> ➢ Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
> ➢ Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin.

> Mono-, Di- und Trialkylphosphate sowie Mono-, Di- und/oder Tri-PEG-alkylphosphate und deren Salze;
> Wollwachsalkohole;
> Polysiloxan-Polyalkyl-Polyether-Copolymere bzw. entsprechende Derivate;
> Polyalkylenglycole sowie
> Glycerincarbonat.

Die **Anlagerungsprodukte von Ethylenoxid und/oder** von **Propylenoxid** an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stöffmengen von Ethylenoxid und/ oder Propylenoxid und Substrat, mit denen die Anlagerungsreaktion durchgeführt wird, entspricht. $C_{12/18}$-Fettsäuremono- und -diester von Anlagerungsprodukten von Ethylenoxid an Glycerin sind aus **DE 2024051 PS** als Rückfettungsmittel für kosmetische Zubereitungen bekannt.

Typische Beispiele für geeignete **Partialglyceride** sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als **Sorbitanester** kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete **Polyglycerinester** sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische.

Beispiele für weitere geeignete **Polyolester** sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete **zwitterionische Tenside** sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer $C_{8/18}$-Alkyl- oder -Acylgruppe im Molekül mindestens eine freie Aminogruppe und mindestens eine -COOH- oder -SO$_3$H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12/18}$-Acylsarcosin.

Schließlich kommen auch **Kationtenside** als Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und - diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte VinylpyrrolidonNinylimidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quatemiertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxyproylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976).**

Typische Beispiele für **Fette** sind Glyceride, als **Wachse** kommen u.a. natürliche Wachse, wie z.B. Candelillawachs, Camaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.

Als **Stabilisatoren** können Metallsalze von Fettsäuren, wie z.B. Magnesium-, Aluminium- und/oder Zinkstearat bzw. -ricinoleat eingesetzt werden.

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Kosmetische **Deodorantien** (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

Als **keimhemmende Mittel** sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonolaurat (GML), Diglycerinmonocaprinat (DMC), Salicylsäure-N-alkylamide wie z. B. Salicylsäure-n-octylamid oder Salicylsäure-n-decylamid.

Als **Enzyminhibitoren** sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung, Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als **Geruchsabsorber** eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, $\alpha$-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilat, Irotyl und Floramat allein oder in Mischungen, eingesetzt.

**Antitranspirantien** (Antiperspirantien) reduzieren durch Beeinflussung der Aktivität der ekkrinen Schweißdrüsen die Schweißbildung, und wirken somit Achselnässe und Körpergeruch entgegen.

Wässrige oder wasserfreie Formulierungen von Antitranspirantien enthalten typischerweise folgende Inhaltsstoffe:

> adstringierende Wirkstoffe,
> Ölkomponenten,
> nichtionische Emulgatoren,
> Coemulgatoren,
> Konsistenzgeber,
> Hilfsstoffe wie z. B. Verdicker oder Komplexierungsmittel und/oder
> nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkoniumpentachlorohydrat und deren Komplexverbindungen z. B. mit Aminosäuren wie Glycin.

Daneben können in Antitranspirantien übliche öllösliche und wasserlösliche Hilfsmittel in geringeren Mengen enthalten sein. Solche öllöslichen Hilfsmittel können z.B. sein:

- ➢ entzündungshemmende, hautschützende oder wohlriechende ätherische Öle,
- ➢ synthetische hautschützende Wirkstoffe und/oder
- ➢ öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

Als **Antischuppenmittel** können Octopirox® (1-Hydroxy-4-methyl-6-(2,4,4-trimythylpentyl)-2-(1H)-pyridon-monoethanolaminsalz), Baypival, Pirocton Olamin, Ketoconazol®, (4-Acetyl-1-{-4-[2-(2.4-dichlorphenyl)r-2-(1H-imidazol-1-ylmethyl)-1,3-dioxylan-c-4-ylmethoxyphenyl}piperazin, Selendisulfid, Schwefel kolloidal, Schwefelpolyehtylenglykolsorbitanmonooleat, Schwefelrizinolpolyehtoxylat, Schwe-felteer Destillate, Salicylsäure (bzw. in Kombination mit Hexachlorophen), Undexylensäure Monoethanolamid Sulfosuccinat Na-Salz, Lamepon® UD (Protein-Undecylensäure-rekondensat, Zinkpyrethion, Aluminiumpyrition und Magnesiumpyrithion / Dipyrithion-Magnesiomsulfat eingesetzt werden.

Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Als **Quellmittel** für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R. Lochhead in **Cosm.Toil. 108, 95 (1993)** entnommen werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:

- ➢ 3-Benzylidencampher bzw. 3-Benzylidennorcampher und dessen Derivate, z.B. 3-(4-Methylbenzyliden)campher wie in der **EP 0693471 B1** beschrieben;
- ➢ 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- ➢ Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- ➢ Ester der Salicylsäure, vorzugsweise Salicylsäure-2-ethylhexylester, Salicylsäure-4-isopropylbenzylester, Salicylsäurehomomenthylester;
- ➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- ➢ Ester der Benzalmalonsäure, vorzugsweise 4-Methoxybenzmalonsäuredi-2-ethylhexylester;
- ➢ Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben oder Dioctyl Butamido Triazone (Uvasorb® HEB);
- ➢ Propan-1,3-dione, wie z.B. 1-(4-tert.Butylphenyl)-3-(4'methoxyphenyl)propan-1,3-dion;
- ➢ Ketotricyclo(5.2.1.0)decan-Derivate, wie in der **EP 0694521 B1** beschrieben.

Als wasserlösliche Substanzen kommen in Frage:

- ➢ 2-Phenylbenzimidazol-5-sulfonsäure und deren Alkali-, Erdalkali-, Ammonium-, Alkylammonium-, Alkanolammonium- und Glucammoniumsalze;
- ➢ Sulfonsäurederivate von Benzophenonen, vorzugsweise 2-Hydroxy-4-methoxybenzophenon-5-sulfonsäure und ihre Salze;
- ➢ Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D, L-Camosin, D-Camosin, L-Carnosin und deren Derivate (z.B. Anserin), Carotinoide, Carotine (z.B. $\alpha$-Carotin, $\beta$-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cho-lesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis $\mu$mol/kg), ferner (Metall)-Chelatoren (z.B. $\alpha$-Hydroxyfettsäuren, Palmitinsäure, Phytinsäure, Lactoferrin), $\alpha$-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. $\gamma$-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, $\alpha$-Glycosylrutin, Ferulasäure, Furfurylidenglucitol, Camosin, Butylhydroxytoluol, Bu-tylhydroxyanisol, Nordihydroguajakharzsäure, Nordihydroguajaretsäure, Trihydroxybutyrophenon, Harnsäure und de-ren Derivate, Mannose und deren Derivate, Superoxid-Dismutase, Zink und dessen Derivate (z.B. ZnO, $ZnSO_4$) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind

- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;

➢ Dialkoholamine, wie Diethanolamin oder 2-Amino-1,3-propandiol.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyl-m-toluamid, 1,2-Pentandiol oder Ethyl Butylacetylaminopropionate in Frage, als **Selbstbräuner** eignet sich Dihydroxyaceton. Als **Tyrosinhinbitoren,** die die Bildung von Melanin verhindern und Anwendung in Depigmentierungsmitteln finden, kommen beispielsweise Arbutin, Kojisäure, Cumarinsäure und Ascorbinsäure (Vitamin C) in Frage.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethem zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilliat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

**Beispiele**

Verschiedene Emulsionen wurden nach der heiß/heiß Phaseninversionsmethode hergestellt und auf ihre Feinteiligkeit untersucht. Die Ergebnisse sind in Tabelle 1 zusammengefaßt. Die Beispiele 1 bis 8 sind erfindungsgemäß, die Beispiele V1 und V2 dienen zum Vergleich. Es zeigt sich, daß die Emulsionen, die die erfindungsgemäße Emulgatormischung enthalten, deutlich feinteiliger als die Vergleichsprodukte sind.

Tabelle 1

| PIT-Emulsionen | | | | | | | | | | |
|---|---|---|---|---|---|---|---|---|---|---|
| Zusammensetzung | 1 | 2 | 3 | 4 | 5 | 6 | 7 | 8 | V1 | V2 |
| **Cegesoft® C24** <br> 2-Ethylhexyl Palmitate | 5,0 | 4,0 | 5,0 | - | - | - | - | - | 5,0 | - |
| **Cetiol® A** <br> Hexyl Laurate | 6,3 | 5,0 | 5,0 | 7,0 | - | - | - | - | 6,3 | 7,0 |

Tabelle 1   (fortgesetzt)

| PIT-Emulsionen | | | | | | | | | | |
|---|---|---|---|---|---|---|---|---|---|---|
| Zusammensetzung | 1 | 2 | 3 | 4 | 5 | 6 | 7 | 8 | V1 | V2 |
| **Cetiol® SN**<br>Cetearyl Isononanoate | 3,76 | 3,0 | 2,0 | 6,0 | - | - | - | - | 3,76 | 6,0 |
| **Cetiol® S**<br>Dioctylcyclohexane | - | - | - | - | 5,0 | 3,0 | - | - | - | - |
| **Cetiol® OE**<br>Dicaprylyl Ether | - | - | - | - | 3,0 | 5,0 | - | - | - | - |
| **Eutanol® G 16**<br>Octyldodecanol | 5,0 | 2,0 | 5,0 | 4,0 | 2,0 | 2,0 | - | - | 5,0 | 4,0 |
| **Myritol® 331**<br>Coco Glycerides | - | - | - | - | - | - | 2,0 | 2,0 | - | - |
| **Paraffin Oil** | - | - | - | - | - | - | 5,0 | 5,0 | - | - |
| **Almond Oil** | - | - | - | - | - | - | 3,0 | 3,0 | - | - |
| **Emulgade® PL 6850**<br>Cetearyl Glucoside (and) Cetearyl Alcohol | 3,3 | 2,2 | 3,6 | 2,0 | 1,6 | 1,5 | 1,6 | 1,6 | 3,3 | 2,0 |
| **Mergital® B 10**<br>Beheneth-10 | 3,3 | 2,2 | 3,6 | 2,0 | 1,6 | 1,5 | 1,6 | 1,6 | - | - |
| **Eumulgin® B2**<br>Ceteareth-20 | - | 0,9 | - | 0,9 | 0,8 | 1,0 | 0,8 | 0,8 | 4,7 | 2,9 |
| **Mergital® B 25**<br>Beheneth-25 | 1,4 | - | 1,3 | - | - | - | - | - | - | - |
| **Wasser** | ad 100 | | | | | | | | | |
| *Anleil Teilchen < 0,2 µm [%]* | 60 | 70 | 92 | 100 | 100 | 96 | 96 | 85 | 41 | 53 |

**Patentansprüche**

1. Kosmetische Emulsionen auf Basis von Wasser, Ölkörpem und einer Emulgatormischung, bei denen mindestens 60 % der Tröpfchen einen mittleren Durchmesser kleiner 0,2 µm aufweisen, enthaltend

(a) $C_{16}$-$C_{22}$-Alkyl- und/oder Alkenyloligoglykoside,
(b) $C_{16}$-$C_{22}$-Fettalkohole und
(c) $C_{22}$-Fettalkoholethoxylate.

2. Emulsionen nach Anspruch 1, **dadurch gekennzeichnet, daß** sie als Komponente (a) Alkylund/oder Alkenyloligoglykoside der Formel **(I)** enthalten,

$$R^1O\text{-}[G]_p$$

**(I)**

in der $R^1$ für einen Alkyl- und/oder Alkenylrest mit 16 bis 22 Kohlenstoffatomen, G für einen Zuckerrest mit 5 oder 6 Kohlenstoffatomen und p für Zahlen von 1 bis 10 steht.

3. Emulsionen nach den Ansprüchen 1 und/oder 2, **dadurch gekennzeichnet, daß** sie als Komponente (a) Glykoside der Formel (I) enthalten, in der $R^1$ für einen Cetearyl- oder einen Behenylrest steht.

4. Emulsionen nach mindestens einem der Ansprüche 1 bis 3, **dadurch gekennzeichnet, daß** sie als Komponente (b) Fettalkohole der Formel **(II)** enthalten,

$$R^2OH$$
$$(II)$$

in der $R^2$ für einen aliphatischen, linearen oder verzweigten Kohlenwasserstoffrest mit 16 bis 22 Kohlenstoffatomen und 0 und/oder 1, 2 oder 3 Doppelbindungen steht.

5. Emulsionen nach mindestens einem der Ansprüche 1 bis 4, **dadurch gekennzeichnet, daß** sie als Komponente (b) Cetearylalkohol oder Behenylalkohol enthalten.

6. Emulsionen nach mindestens einem der Ansprüche 1 bis 5, **dadurch gekennzeichnet, daß** die Komponenten (a) und (b) gleiche Fettreste aufweisen.

7. Emulsionen nach mindestens einem der Ansprüche 1 bis 6, **dadurch gekennzeichnet, daß** sie als Komponente (c) Fettalkoholethoxylate der Formel **(III)** enthalten,

$$R^3O(CH_2CH_2O)_nH$$
$$(III)$$

in der $R^3$ für einen Behenylrest und n für Zahlen von 1 bis 15 steht.

8. Emulsionen nach mindestens einem der Ansprüche 1 bis 7, **dadurch gekennzeichnet, daß** sie als fakultative Komponente (d) weiterhin hydrophile Fettalkoholethoxylate der Formel **(IV)** enthalten,

$$R^4O(CH_2CH_2O)_mH$$
$$(IV)$$

in der $R^4$ für einen linearen oder verzweigten Alkyl- und/oder Alkenylrest mit 6 bis 22 Kohlenstoffatomen und m für Zahlen von 15 bis 35 steht.

9. Emulsionen nach mindestens einem der Ansprüche 1 bis 8, **dadurch gekennzeichnet, daß** sie

(a) 1 bis 50 Gew.-% Ölkörper und
(b) 1 bis 25 Gew.-% Emulgatormischung

mit der Maßgabe enthalten, daß sich die Mengenangaben mit Wasser und gegebenenfalls weiteren üblichen Hilfs- und Zusatzstoffen zu 100 Gew.-% ergänzen.

10. Emulsionen nach mindestens einem der Ansprüche 1 bis 9, **dadurch gekennzeichnet, daß** sie

(a) 1 bis 50 Gew.-% Ölkörper,
(b1) 1 bis 5 Gew.-% $C_{16}$-$C_{22}$-Alkyl- und/oder Alkenyloligoglykoside,
(b2) 1 bis 5 Gew.-% $C_{16}$-$C_{22}$-Fettalkohole,
(b3) 1 bis 5 Gew.-% $C_{22}$-Fettalkoholethoxylate und
(b4) 0 bis 4 Gew.-% hydrophile Fettalkoholethoxylate

mit der Maßgabe enthalten, daß sich die Mengenangaben mit Wasser und gegebenenfalls weiteren üblichen Hilfs- und Zusatzstoffen zu 100 Gew.-% ergänzen.

**EP 1 189 577 B1**

**Claims**

1. Cosmetic emulsions based on water, oil components and an emulsifier mixture where at least 60% of the droplets have a mean diameter of less than 0.2 μm, containing

   (a) $C_{16-22}$ alkyl and/or alkenyl oligoglycosides,
   (b) $C_{16-22}$ fatty alcohols and
   (c) $C_{22}$ fatty alcohol ethoxylates.

2. Emulsions as claimed in claim 1, **characterized in that** they contain alkyl and/or alkenyl oligoglycosides corresponding to formula **(I):**

$$R^1O\text{-}[G]_p$$

$$(I)$$

where $R^1$ is an alkyl and/or alkenyl group containing 16 to 22 carbon atoms, G is a sugar unit containing 5 or 6 carbon atoms and p is a number of 1 to 10, as component (a).

3. Emulsions as claimed in claims 1 and/or 2, **characterized in that** they contain glycosides of formula (I), where $R^1$ is a cetearyl or behenyl group, as component (a).

4. Emulsions as claimed in at least one of claims 1 to 3, **characterized in that** they contain fatty alcohols corresponding to formula **(II):**

$$R^2OH$$

$$(II)$$

where $R^2$ is an aliphatic, linear or branched hydrocarbon radical containing 16 to 22 carbon atoms and 0 and/or 1, 2 or 3 double bonds, as component (b).

5. Emulsions as claimed in at least one of claims 1 to 4, **characterized in that** they contain cetearyl alcohol or behenyl alcohol as component (b).

6. Emulsions as claimed in at least one of claims 1 to 5, **characterized in that** components (a) and (b) have the same fatty residues.

7. Emulsions as claimed in at least one of claims 1 to 6, **characterized in that** they contain fatty alcohol ethoxylates corresponding to formula **(III):**

$$R^3O(CH_2CH_2O)_nH$$

$$(III)$$

where $R^3$ is a behenyl radical and n is a number of 1 to 15, as component (c).

8. Emulsions as claimed in at least one of claims 1 to 7, **characterized in that** they contain hydrophilic fatty alcohol ethoxylates corresponding to formula **(IV):**

14

$$R^4O(CH_2CH_2O)_mH$$
$$(IV)$$

where $R^4$ is a linear or branched alkyl and/or alkenyl group containing 6 to 22 carbon atoms and m is a number of 15 to 35,
as an optional component (d).

9. Emulsions as claimed in at least one of claims 1 to 8, **characterized in that** they contain

(a) 1 to 50% by weight of oil component and
(b) 1 to 25% by weight of emulsifier mixture,

with the proviso that the quantities shown add up to 100% by weight with water and optionally other typical auxiliaries and additives.

10. Emulsions as claimed in at least one of claims 1 to 9, **characterized in that** they contain

(a) 1 to 50% by weight of oil component,
(b1) 1 to 5% by weight of $C_{16-22}$ alkyl and/or alkenyl oligoglycosides,
(b2) 1 to 5% by weight of $C_{16-22}$ fatty alcohols,
(b3) 1 to 5% by weight of $C_{22}$ fatty alcohol ethoxylates and
(b4) 0 to 4% by weight of hydrophilic fatty alcohol ethoxylates,

with the proviso that the quantities shown add up to 100% by weight with water and optionally other typical auxiliaries and additives.

**Revendications**

1. Emulsions cosmétiques à base d'eau, de composés huileux et d'un mélange d'agents émulsionnants, dans lesquelles au moins 60 % des gouttelettes possèdent un diamètre moyen inférieur à 0,2 μm, contenant :

a) des (alkyl- et/ou alkényl en $C_{16}$ à $C_{22}$) oligoglycosides,
b) des alcools gras en $C_{16}$ à $C_{22}$ et
c) des éthoxylates d'alcools gras en $C_{22}$.

2. Emulsions selon la revendication 1,
**caractérisées en ce qu'**
elles renferment comme composant a) des alkyl et/ou des alkényloligoglycosides de formule (I)

$$R^1O-[G]_p \hspace{4cm} (I)$$

dans laquelle $R^1$ représente un reste alkyle et/ou alkényle ayant de 16 à 22 atomes de carbone, G représente un reste de sucre ayant 5 ou 6 atomes de carbone et p représenté des nombres allant de 1 à 10.

3. Emulsions selon les revendications 1 et/ou 2,
**caractérisées en ce qu'**
elles renferment comme composant a) un glycoside de formule (I) dans laquelle $R^1$ représente un reste cétéaryle ou un reste béhényle.

4. Emulsions selon au moins une des revendications 1 à 3,
**caractérisées en ce qu'**
elles renferment comme composant b) des alcools gras de formule (II)

$$R^2OH \qquad\qquad (II)$$

dans laquelle $R^2$ représente un reste hydrocarboné aliphatique, linéaire ou ramifié ayant de 16 à 22 atomes de carbone, et 0 et/ou 1, 2 ou 3 doubles liaisons.

**5.** Emulsions selon au moins une des revendications 1 à 4,
**caractérisées en ce qu'**
elles renferment comme composant b) de l'alcool cétéarylique ou de l'alcool béhénique.

**6.** Emulsions selon au moins une des revendications 1 à 5,
**caractérisées en ce que**
les composants (a) et (b) possèdent les mêmes restes gras.

**7.** Emulsions selon au moins l'une des revendications 1 à 6,
**caractérisées en ce qu'**
elles renferment comme composant c) des éthoxylates d'alcool gras de formule (III)

$$R^3O(CH_2CH_2O)_nH \qquad\qquad (III)$$

dans laquelle $R^3$ représente un reste béhényle et n représente des nombres allant de 1 à 15.

**8.** Emulsions selon au moins l'une des revendications 1 à 7,
**caractérisées en ce qu'**
elles renferment en outre comme composant facultatif (d) des éthoxylates d'alcool gras hydrophiles de formule (IV)

$$R^4O(CH_2CH_2O)_mH \qquad\qquad (IV)$$

dans laquelle $R^4$ représente un reste alkyle et/ou alkényle linéaire ou ramifié ayant de 6 à 22 atomes de carbone et m représente des nombres allant de 15 à 35.

**9.** Emulsions selon au moins l'une des revendications 1 à 8,
**caractérisées en ce qu'**
elles renferment

    a) de 1 à 50 % en poids de composé huileux et
    b) de 1 à 25 % en poids de mélange d'agents émulsionnants

avec la précision que les données en quantités se complètent avec de l'eau et éventuellement d'autres adjuvants et additifs usuels à 100 % en poids.

**10.** Emulsions selon au moins l'une des revendications 1 à 9,
**caractérisées en ce qu'**
elles renferment

    a) de 1 à 50 % en poids de composé huileux,
    b1) de 1 à 5 % en poids d'(alkyl et/ou alkényle en $C_{16}$ à $C_{22}$) oligoglycosides,
    b2) de 1 à 5 % en poids d'alcools gras en $C_{16}$-$C_{22}$,
    b3) de 1 à 5 % en poids d'éthoxylates d'alcools gras en $C_{22}$ et
    b4) de 0 à 4 % en poids d'éthoxylates d'alcool gras hydrophiles

avec la précision que les données en quantités se complètent avec de l'eau et éventuellement d'autres adjuvants et additifs usuels, à 100 % en poids.